# EUROPEAN PATENT APPLICATION

(11) **EP 3 816 292 A1**
(43) Date of publication of application: **05.05.2021**
(21) Application number: 19830583.1
(22) Date of filing: 03.07.2019
(51) Int. Cl.: C12N 15/29, C12N 15/82, A01H 5/12, A01H 6/54

(54) **METHOD OF OBTAINING MULTI-LEAF ALFALFA MATERIAL BY MEANS OF MSPALM1 ARTIFICIAL SITE-DIRECTED MUTANT**

(30) Priority: 04.07.2018 CN 201810724563
(71) Applicant: Guangdong Sanjie Forage Biotechnology Co., Ltd, Guangzhou, Guangdong 510623 (CN)
(72) Inventor: CHEN, Haitao, Guangzhou, Guangdong 510507 (CN); WANG, Wen, Guangzhou, Guangdong 510507 (CN); XIE, Xiongping, Guangzhou, Guangdong 510507 (CN); QIU, Qiang, Guangzhou, Guangdong 510507 (CN); SHANG, Zhanhuan, Guangzhou, Guangdong 510507 (CN); SU, Kexian, Guangzhou, Guangdong 510507 (CN); HE, Hui, Guangzhou, Guangdong 510507 (CN)
(74) Representative: Sonn & Partner Patentanwälte
(86) International application number: PCT/CN2019/094632
(87) International publication number: WO 2020/007332

(57) **Abstract**

Disclosed is a method for obtaining multileaflet *Medicago sativa* materials by means of *MsPALM1* artificial site-directed mutants. The method comprises: selecting a target site from an exon region of a compound leaf developmental regulatory gene *MsPALM1* of *Medicago sativa* and constructing a plant CRISPR/Cas9 editing recombinant vector MsCRISPR/Cas9::*PALM1* and introducing the vector into *Medicago sativa* cells and regenerating into plants, cutting and repairing to cause a loss-of-function mutation in the *MsPALM1* gene of *Medicago sativa* cells, and then screening the mutant plants by restriction endonuclease digestion and/or targeted deep sequencing of the target sites of the regenerated plantsgenome to obtain lines carrying four *MsPALM1* allelic genes with simultaneous loss of function mutation. After phenotypic identification, it was confirmed that the compound leaves of the regenerated plants changed from three leaflets to five leaflets. The method can quickly obtain multileaflet *Medicago sativa* materials with a short breeding period and stable trait.

SEQUENCE LISTING

<110>    GUANGDONG SANJIE FORAGE BIOTECHNOLOGY CO., LTD

<120> Method of obtaining multileaflet Medicago sativa materials by means of
MsPALM1 artificial site-directed mutants

<130> GWPCTP202011371

<160> 2

<170> PatentIn version 3.5

<210> 1
<211> 615
<212> DNA
<213> artificial sequence

<220>
<223> sgRNA expression cassette

<400> 1
atccaacatt tcacttgagt taactcaata gcaagaataa cgtccatagt ttcagcattc 60

aagcaaaacg gccaagaaaa tcagcttggt aatttcagtg agacctggac taccataagc 120

agcaccgcct attacactta atggggtaaa gtaaaacgag ccacatcacc tccttgattt 180

taaggagcat ttgaaggagt ataaaaagaa tgtatgtaat gtaaggttgt gttgtgtcat 240

tcaagatagc aagacggacc aaagcttcta tgtatctatc tatgtctatg atatgatgat 300

tgtattgatt tggtttgagt acagtgaggg agagggagga acttcttcac ttgtttattt 360

aacctgaaac tcaactcaaa tcactgagag tgaatgttga gaaataagta ttatgttatg 420

tttgctttgc tattagtccc acatcgctta catatacttc agttatattg tttatatagc 480

ctagacgaac agcagggttt ggctcgcagg tgaacacaac acctgcacac gttttagagc 540

tagaaatagc aagttaaaat aaggctagtc cgttatcaac ttgaaaaagt ggcaccgagt 600

cggtgctttt ttttt 615

<210> 2
<211> 5048
<212> DNA
<213> artificial sequence

<220>
<223> Cas9 nuclease expression cassette

<400> 2
tgagactttt caacaaaggg tgatatccgg aaacctcctc ggattccatt gcccagctat 60

ctgtcacttt attgtgaaga tagtggaaaa ggaaggtggc tcctacaaat gccatcattg 120

cgataaagga aaggccatcg ttgaagatgc ctctgccgac agtggtccca aagatggacc 180

cccacccacg aggagcatcg tggaaaaaga agacgttcca accacgtctt caaagcaagt 240

ggattgatgt gatatctcca ctgacgtaag ggatgacgca caatcccact atccttcgca 300

agacccttcc tctatataag gaagttcatt tcatttggag aggacctcga cctcaacaca 360

acatatacaa aacaaacgaa tctcaagcaa tcaagcattc tacttctatt gcagcaattt 420

aaatcatttc ttttaaagca aaagcaattt tctgaaaatt ttcaccattt acgaacgata 480

ctcgagatgg actataagga ccacgacgga gactacaagg atcatgatat tgattacaaa 540

gacgatgacg ataagatggc cccaaagaag aagcggaagg tcggtatcca cggagtccca 600

gcagccgaca agaagtacag catcggcctg gacatcggca ccaactctgt gggctgggcc 660

gtgatcaccg acgagtacaa ggtgcccagc aagaaattca aggtgctggg caacaccgac 720

cggcacagca tcaagaagaa cctgatcgga gccctgctgt tcgacagcgg cgaaacagcc 780

gaggccaccc ggctgaagag aaccgccaga agaagataca ccagacggaa gaaccggatc 840

tgctatctgc aagagatctt cagcaacgag atggccaagg tggacgacag cttcttccac 900

agactggaag agtccttcct ggtggaagag gataagaagc acgagcggca ccccatcttc 960

ggcaacatcg tggacgaggt ggcctaccac gagaagtacc ccaccatcta ccacctgaga 1020

aagaaactgg tggacagcac cgacaaggcc gacctgcggc tgatctatct ggccctggcc 1080

cacatgatca agttccgggg ccacttcctg atcgagggcg acctgaaccc cgacaacagc 1140

gacgtggaca agctgttcat ccagctggtg cagacctaca accagctgtt cgaggaaaac 1200

cccatcaacg ccagcggcgt ggacgccaag gccatcctgt ctgccagact gagcaagagc 1260

agacggctgg aaaatctgat cgcccagctg cccggcgaga agaagaatgg cctgttcgga 1320

aacctgattg ccctgagcct gggcctgacc cccaacttca agagcaactt cgacctggcc 1380

gaggatgcca aactgcagct gagcaaggac acctacgacg acgacctgga caacctgctg 1440

gcccagatcg gcgaccagta cgccgacctg tttctggccg ccaagaacct gtccgacgcc 1500

atcctgctga gcgacatcct gagagtgaac accgagatca ccaaggcccc cctgagcgcc 1560

tctatgatca agagatacga cgagcaccac caggacctga ccctgctgaa agctctcgtg 1620

cggcagcagc tgcctgagaa gtacaaagag attttcttcg accagagcaa gaacggctac 1680

gccggctaca ttgacggcgg agccagccag gaagagttct acaagttcat caagcccatc 1740

ctggaaaaga tggacggcac cgaggaactg ctcgtgaagc tgaacagaga ggacctgctg 1800

cggaagcagc ggaccttcga caacggcagc atcccccacc agatccacct gggagagctg 1860

cacgccattc tgcggcggca ggaagatttt tacccattcc tgaaggacaa ccgggaaaag 1920

atcgagaaga tcctgacctt ccgcatcccc tactacgtgg gccctctggc caggggaaac 1980

agcagattcg cctggatgac cagaaagagc gaggaaacca tcaccccctg gaacttcgag 2040

gaagtggtgg acaagggcgc ttccgcccag agcttcatcg agcggatgac caacttcgat 2100

aagaacctgc ccaacgagaa ggtgctgccc aagcacagcc tgctgtacga gtacttcacc 2160

gtgtataacg agctgaccaa agtgaaatac gtgaccgagg gaatgagaaa gcccgccttc 2220

ctgagcggcg agcagaaaaa ggccatcgtg gacctgctgt tcaagaccaa ccggaaagtg 2280

accgtgaagc agctgaaaga ggactacttc aagaaaatcg agtgcttcga ctccgtggaa 2340

atctccggcg tggaagatcg gttcaacgcc tccctgggca cataccacga tctgctgaaa 2400

attatcaagg acaaggactt cctggacaat gaggaaaacg aggacattct ggaagatatc 2460

gtgctgaccc tgacactgtt tgaggacaga gagatgatcg aggaacggct gaaaacctat 2520

gcccacctgt tcgacgacaa agtgatgaag cagctgaagc ggcggagata caccggctgg 2580

ggcaggctga gccggaagct gatcaacggc atccgggaca agcagtccgg caagacaatc 2640

ctggatttcc tgaagtccga cggcttcgcc aacagaaact tcatgcagct gatccacgac 2700

gacagcctga cctttaaaga ggacatccag aaagcccagg tgtccggcca gggcgatagc 2760

ctgcacgagc acattgccaa tctggccggc agccccgcca ttaagaaggg catcctgcag 2820

acagtgaagg tggtggacga gctcgtgaaa gtgatgggcc ggcacaagcc cgagaacatc 2880

gtgatcgaaa tggccagaga gaaccagacc acccagaagg gacagaagaa cagccgcgag 2940

agaatgaagc ggatcgaaga gggcatcaaa gagctgggca gccagatcct gaaagaacac 3000

cccgtggaaa acacccagct gcagaacgag aagctgtacc tgtactacct gcagaatggg 3060

cgggatatgt acgtggacca ggaactggac atcaaccggc tgtccgacta cgatgtggac 3120

catatcgtgc ctcagagctt tctgaaggac gactccatcg acaacaaggt gctgaccaga 3180

agcgacaaga accggggcaa gagcgacaac gtgccctccg aagaggtcgt gaagaagatg 3240

aagaactact ggcggcagct gctgaacgcc aagctgatta cccagagaaa gttcgacaat 3300

ctgaccaagg ccgagagagg cggcctgagc gaactggata aggccggctt catcaagaga 3360

cagctggtgg aaacccggca gatcacaaag cacgtggcac agatcctgga ctcccggatg 3420

aacactaagt acgacgagaa tgacaagctg atccgggaag tgaaagtgat caccctgaag 3480

tccaagctgg tgtccgattt ccggaaggat ttccagtttt acaaagtgcg cgagatcaac 3540

aactaccacc acgcccacga cgcctacctg aacgccgtcg tgggaaccgc cctgatcaaa 3600

aagtacccta agctggaaag cgagttcgtg tacggcgact acaaggtgta cgacgtgcgg 3660

aagatgatcg ccaagagcga gcaggaaatc ggcaaggcta ccgccaagta cttcttctac 3720

agcaacatca tgaacttttt caagaccgag attaccctgg ccaacggcga gatccggaag 3780

cggcctctga tcgagacaaa cggcgaaacc ggggagatcg tgtgggataa gggccgggat 3840

tttgccaccg tgcggaaagt gctgagcatg ccccaagtga atatcgtgaa aaagaccgag 3900

gtgcagacag gcggcttcag caaagagtct atcctgccca agaggaacag cgataagctg 3960

atcgccagaa agaaggactg ggaccctaag aagtacggcg gcttcgacag ccccaccgtg 4020

gcctattctg tgctggtggt ggccaaagtg gaaaagggca agtccaagaa actgaagagt 4080

gtgaaagagc tgctggggat caccatcatg gaaagaagca gcttcgagaa gaatcccatc 4140

gactttctgg aagccaaggg ctacaaagaa gtgaaaaagg acctgatcat caagctgcct 4200

aagtactccc tgttcgagct ggaaaacggc cggaagagaa tgctggcctc tgccggcgaa 4260

ctgcagaagg gaaacgaact ggccctgccc tccaaatatg tgaacttcct gtacctggcc 4320

agccactatg agaagctgaa gggctccccc gaggataatg agcagaaaca gctgtttgtg 4380

gaacagcaca agcactacct ggacgagatc atcgagcaga tcagcgagtt ctccaagaga 4440

gtgatcctgg ccgacgctaa tctggacaaa gtgctgtccg cctacaacaa gcaccgggat 4500

aagcccatca gagagcaggc cgagaatatc atccacctgt ttaccctgac caatctggga 4560

gcccctgccg ccttcaagta ctttgacacc accatcgacc ggaagaggta caccagcacc 4620

aaagaggtgc tggacgccac cctgatccac cagagcatca ccggcctgta cgagacacgg 4680

atcgacctgt ctcagctggg aggcgacaaa aggccggcgg ccacgaaaaa ggccggccag 4740

gcaaaaaaga aaaagtaagg atcctgattg atcgatagag ctcgaatttc cccgatcgtt 4800

caaacatttg gcaataaagt ttcttaagat tgaatcctgt tgccggtctt gcgatgatta 4860

tcatataatt tctgttgaat tacgttaagc atgtaataat taacatgtaa tgcatgacgt 4920

tatttatgag atgggttttt atgattagag tcccgcaatt atacatttaa tacgcgatag 4980

aaaacaaaat atagcgcgca aactaggata aattatcgcg cgcggtgtca tctatgttac 5040

tagatcgg 5048

SEQUENCE LISTING

<110>  GUANGDONG SANJIE FORAGE BIOTECHNOLOGY CO., LTD

<120>  Method of obtaining multileaflet Medicago sativa materials by means
of MsPALM1 artificial site-directed mutants

<130>  GWPCTP202011371

<160>  2

<170>  PatentIn version 3.5

<210>  1
<211>  615
<212>  DNA
<213>  artificial sequence

<220>
<223>  sgRNA expression cassette

<400>  1
atccaacatt tcacttgagt taactcaata gcaagaataa cgtccatagt ttcagcattc      60

aagcaaaacg gccaagaaaa tcagcttggt aatttcagtg agacctggac taccataagc     120

agcaccgcct attacactta atggggtaaa gtaaaacgag ccacatcacc tccttgattt     180

taaggagcat ttgaaggagt ataaaaagaa tgtatgtaat gtaaggttgt gttgtgtcat     240

tcaagatagc aagacggacc aaagcttcta tgtatctatc tatgtctatg atatgatgat     300

tgtattgatt tggtttgagt acagtgaggg agagggagga acttcttcac ttgtttattt     360

aacctgaaac tcaactcaaa tcactgagag tgaatgttga gaaataagta ttatgttatg     420

tttgctttgc tattagtccc acatcgctta catatacttc agttatattg tttatatagc     480

ctagacgaac agcagggttt ggctcgcagg tgaacacaac acctgcacac gttttagagc     540

tagaaatagc aagttaaaat aaggctagtc cgttatcaac ttgaaaaagt ggcaccgagt     600

cggtgctttt ttttt                                                      615

<210>  2
<211>  5048
<212>  DNA
<213>  artificial sequence

<220>
<223>  Cas9 nuclease expression cassette

<400>  2
tgagactttt caacaaaggg tgatatccgg aaacctcctc ggattccatt gcccagctat      60

ctgtcacttt attgtgaaga tagtggaaaa ggaaggtggc tcctacaaat gccatcattg     120

cgataaagga aaggccatcg ttgaagatgc ctctgccgac agtggtccca aagatggacc     180

cccacccacg aggagcatcg tggaaaaaga agacgttcca accacgtctt caaagcaagt     240

ggattgatgt gatatctcca ctgacgtaag ggatgacgca caatcccact atccttcgca     300

agacccttcc tctatataag gaagttcatt tcatttggag aggacctcga cctcaacaca 360

acatatacaa aacaaacgaa tctcaagcaa tcaagcattc tacttctatt gcagcaattt 420

aaatcatttc ttttaaagca aaagcaattt tctgaaaatt ttcaccattt acgaacgata 480

ctcgagatgg actataagga ccacgacgga gactacaagg atcatgatat tgattacaaa 540

gacgatgacg ataagatggc cccaaagaag aagcggaagg tcggtatcca cggagtccca 600

gcagccgaca agaagtacag catcggcctg gacatcggca ccaactctgt gggctgggcc 660

gtgatcaccg acgagtacaa ggtgcccagc aagaaattca aggtgctggg caacaccgac 720

cggcacagca tcaagaagaa cctgatcgga gccctgctgt tcgacagcgg cgaaacagcc 780

gaggccaccc ggctgaagag aaccgccaga agaagataca ccagacggaa gaaccggatc 840

tgctatctgc aagagatctt cagcaacgag atggccaagg tggacgacag cttcttccac 900

agactggaag agtccttcct ggtggaagag gataagaagc acgagcggca ccccatcttc 960

ggcaacatcg tggacgaggt ggcctaccac gagaagtacc ccaccatcta ccacctgaga 1020

aagaaactgg tggacagcac cgacaaggcc gacctgcggc tgatctatct ggccctggcc 1080

cacatgatca agttccgggg ccacttcctg atcgagggcg acctgaaccc cgacaacagc 1140

gacgtggaca agctgttcat ccagctggtg cagacctaca accagctgtt cgaggaaaac 1200

cccatcaacg ccagcggcgt ggacgccaag gccatcctgt ctgccagact gagcaagagc 1260

agacggctgg aaaatctgat cgcccagctg cccggcgaga agaagaatgg cctgttcgga 1320

aacctgattg ccctgagcct gggcctgacc cccaacttca agagcaactt cgacctggcc 1380

gaggatgcca aactgcagct gagcaaggac acctacgacg acgacctgga caacctgctg 1440

gcccagatcg gcgaccagta cgccgacctg tttctggccg ccaagaacct gtccgacgcc 1500

atcctgctga gcgacatcct gagagtgaac accgagatca ccaaggcccc cctgagcgcc 1560

tctatgatca agagatacga cgagcaccac caggacctga ccctgctgaa agctctcgtg 1620

cggcagcagc tgcctgagaa gtacaaagag attttcttcg accagagcaa gaacggctac 1680

gccggctaca ttgacggcgg agccagccag gaagagttct acaagttcat caagcccatc 1740

ctggaaaaga tggacggcac cgaggaactg ctcgtgaagc tgaacagaga ggacctgctg 1800

cggaagcagc ggaccttcga caacggcagc atcccccacc agatccacct gggagagctg 1860

cacgccattc tgcggcggca ggaagatttt tacccattcc tgaaggacaa ccgggaaaag 1920

atcgagaaga tcctgacctt ccgcatcccc tactacgtgg gccctctggc caggggaaac 1980

agcagattcg cctggatgac cagaaagagc gaggaaacca tcaccccctg gaacttcgag 2040

gaagtggtgg acaagggcgc ttccgcccag agcttcatcg agcggatgac caacttcgat 2100

aagaacctgc ccaacgagaa ggtgctgccc aagcacagcc tgctgtacga gtacttcacc 2160

gtgtataacg agctgaccaa agtgaaatac gtgaccgagg gaatgagaaa gcccgccttc 2220

ctgagcggcg agcagaaaaa ggccatcgtg gacctgctgt tcaagaccaa ccggaaagtg 2280

accgtgaagc agctgaaaga ggactacttc aagaaaatcg agtgcttcga ctccgtggaa 2340

atctccggcg tggaagatcg gttcaacgcc tccctgggca cataccacga tctgctgaaa 2400

attatcaagg acaaggactt cctggacaat gaggaaaacg aggacattct ggaagatatc 2460

gtgctgaccc tgacactgtt tgaggacaga gagatgatcg aggaacggct gaaaacctat 2520

gcccacctgt tcgacgacaa agtgatgaag cagctgaagc ggcggagata caccggctgg 2580

ggcaggctga gccggaagct gatcaacggc atccgggaca agcagtccgg caagacaatc 2640

ctggatttcc tgaagtccga cggcttcgcc aacagaaact tcatgcagct gatccacgac 2700

gacagcctga cctttaaaga ggacatccag aaagcccagg tgtccggcca gggcgatagc 2760

ctgcacgagc acattgccaa tctggccggc agccccgcca ttaagaaggg catcctgcag 2820

acagtgaagg tggtggacga gctcgtgaaa gtgatgggcc ggcacaagcc cgagaacatc 2880

gtgatcgaaa tggccagaga gaaccagacc acccagaagg gacagaagaa cagccgcgag 2940

agaatgaagc ggatcgaaga gggcatcaaa gagctgggca gccagatcct gaaagaacac 3000

cccgtggaaa acacccagct gcagaacgag aagctgtacc tgtactacct gcagaatggg 3060

cgggatatgt acgtggacca ggaactggac atcaaccggc tgtccgacta cgatgtggac 3120

catatcgtgc ctcagagctt tctgaaggac gactccatcg acaacaaggt gctgaccaga 3180

agcgacaaga accggggcaa gagcgacaac gtgccctccg aagaggtcgt gaagaagatg 3240

aagaactact ggcggcagct gctgaacgcc aagctgatta cccagagaaa gttcgacaat 3300

ctgaccaagg ccgagagagg cggcctgagc gaactggata aggccggctt catcaagaga 3360

cagctggtgg aaacccggca gatcacaaag cacgtggcac agatcctgga ctcccggatg 3420

aacactaagt acgacgagaa tgacaagctg atccgggaag tgaaagtgat caccctgaag 3480

tccaagctgg tgtccgattt ccggaaggat ttccagtttt acaaagtgcg cgagatcaac 3540

aactaccacc acgcccacga cgcctacctg aacgccgtcg tgggaaccgc cctgatcaaa 3600

aagtacccta agctggaaag cgagttcgtg tacggcgact acaaggtgta cgacgtgcgg 3660

aagatgatcg ccaagagcga gcaggaaatc ggcaaggcta ccgccaagta cttcttctac 3720

agcaacatca tgaacttttt caagaccgag attaccctgg ccaacggcga gatccggaag 3780

cggcctctga tcgagacaaa cggcgaaacc ggggagatcg tgtgggataa gggccgggat 3840

tttgccaccg tgcggaaagt gctgagcatg ccccaagtga atatcgtgaa aaagaccgag 3900

gtgcagacag gcggcttcag caaagagtct atcctgccca agaggaacag cgataagctg 3960

atcgccagaa agaaggactg ggaccctaag aagtacggcg gcttcgacag ccccaccgtg 4020

gcctattctg tgctggtggt ggccaaagtg gaaaagggca agtccaagaa actgaagagt 4080

gtgaaagagc tgctggggat caccatcatg gaaagaagca gcttcgagaa gaatcccatc 4140

gactttctgg aagccaaggg ctacaaagaa gtgaaaaagg acctgatcat caagctgcct 4200

aagtactccc tgttcgagct ggaaaacggc cggaagagaa tgctggcctc tgccggcgaa 4260

ctgcagaagg gaaacgaact ggccctgccc tccaaatatg tgaacttcct gtacctggcc 4320

agccactatg agaagctgaa gggctccccc gaggataatg agcagaaaca gctgtttgtg 4380

gaacagcaca agcactacct ggacgagatc atcgagcaga tcagcgagtt ctccaagaga 4440

gtgatcctgg ccgacgctaa tctggacaaa gtgctgtccg cctacaacaa gcaccgggat 4500

aagcccatca gagagcaggc cgagaatatc atccacctgt ttaccctgac caatctggga 4560

gcccctgccg ccttcaagta ctttgacacc accatcgacc ggaagaggta caccagcacc 4620

aaagaggtgc tggacgccac cctgatccac cagagcatca ccggcctgta cgagacacgg 4680

atcgacctgt ctcagctggg aggcgacaaa aggccggcgg ccacgaaaaa ggccggccag 4740

gcaaaaaaga aaaagtaagg atcctgattg atcgatagag ctcgaatttc cccgatcgtt 4800

caaacatttg gcaataaagt ttcttaagat tgaatcctgt tgccggtctt gcgatgatta 4860

tcatataatt tctgttgaat tacgttaagc atgtaataat taacatgtaa tgcatgacgt 4920

tatttatgag atgggttttt atgattagag tcccgcaatt atacatttaa tacgcgatag 4980

aaaacaaaat atagcgcgca aactaggata aattatcgcg cgcggtgtca tctatgttac 5040

tagatcgg 5048

## Description

### TECHNICAL FIELD

The present disclosure belongs to the technical field of biological breeding of *Medicago sativa,* and specifically pertains to a method of obtaining multileaflet *Medicago sativa* materials by means of *MsPALM1* artificial site-directed mutants.

### BACKGROUD

Alfalfa (*Medicago sativa* L.) is perennial high-quality legume forage with a long history of cultivation, with the characteristics of strong adaptability, high nutritional value, wide planting area, complete ecological functions and high economic benefits. As a perennial legume forage crop, it has the largest planting area in the world and is reputed as the king of forage. *Medicago sativa* has been cultivated for more than two thousand years in China. At present, the land area for planting *Medicago sativa* in China is over about 1.467 million hectares. The current level of production: the average hay yield is 13-17 t/hm², and the average seed yield is 375-600 kg/hm². Therefore, the grass industry in China is mainly the industrialization of *Medicago sativa,* which has a very important value and status in the development of agriculture and animal husbandry. It is of great significance to cultivate the varieties with high grass yield and protein content to promote the high-quality forage production and the development of animal husbandry in China.

The protein content of *Medicago sativa* is mainly concentrated in leaves, and the trait of leaves is related to the trait of yield. The leaves of *Medicago sativa* are generally ternate compound leaves. The detection data shows that, 60-70% of crude protein and 90% of vitamin are found in leaves of *Medicago sativa,* so high-quality *Medicago sativa* should be leafy. It is therefore determined that our breeding target is cultivating novel varieties with multileaflet, that is, to generate multileaflet *Medicago sativa* varieties with more than 3 leaflets per compound leave through mutation. The multileaflet trait of *Medicago sativa* is considered to have the potential for high yield and good quality. It is very important to cultivate high-quality *Medicago sativa* with multileaflet trait for the development and utilization of high-quality *Medicago sativa.* The multileaflet trait of *Medicago sativa* is also found in nature. Results of previous researches have shown that compared with trifoliolate *Medicago sativa,* the photosynthetic efficiency, the specific leaf weight, the stem-leaf ratio and other growth characteristics of multileaflet *Medicago sativa* are significantly enhanced; additionally, the multileaflet trait of *Medicago sativa* can increase the leaf area of *Medicago sativa,* which is positively correlated with the grass yield of *Medicago sativa;* the grass yield of multileaflet *Medicago sativa* lines is significantly higher than those of trifoliolate *Medicago sativa* varieties; however, the multileaflet trait of *Medicago sativa* are unstable and there is a recessive character of the multileaflet trait of *Medicago sativa.*

Although multileaflet material lines have broad application prospects in the cultivation of new high-quality *Medicago sativa* varieties, there are limited natural genetic mutation resources, and *Medicago sativa* is an autotetraploid plant that is cross-pollinated and has the characteristic of self-incompatibility. Therefore, it is not feasible to obtain a novel homozygous genetically stable *Medicago sativa* variety by traditional hybridization techniques, which has a long breeding period. It is known through searching that Patent No. CN104737899B - A method of breeding multileaflet *Medicago sativa* by space mutagenesis had bred the first new multileaflet *Medicago sativa* variety by space mutagenesis, but this method had the disadvantages of high cost, strict condition requirements, multi-generation hybrid breeding, repeated screening, instability, long time consuming and low repeatability.

In the study of *Medicago truncatula,* a proximal species of *Medicago sativa,* we found that the major gene that regulates the development of leaf primordium is *PALM1* gene which is a single-copy gene with a single exon, and the recessive homozygous mutant of this gene has pentatrinate compound leaves. Using an efficient site-directed genome editing technology to silence four alleles of *MsPALM1* of *Medicago sativa* simultaneously may rapidly generate a new genetically stable *Medicago sativa* variety with multileaflet trait. Therefore, we hope to obtain a method of obtaining multileaflet *Medicago sativa* materials by means of artificial site-directed mutants of a *Medicago sativa* compound leaf developmental regulatory gene *MsPALM1,* by which the four alleles of *MsPALM1* of *Medicago sativa* can be silenced simultaneously and rapid orientation can be achieved to obtain multileaflet *Medicago sativa* materials.

### SUMMARY

With respect to the above problems, the present disclosure provides a method of obtaining multileaflet *Medicago sativa* materials by means of *MsPALM1* artificial site-directed mutants, the method comprises the following steps:
Step (1), Selecting a target site in an exon region of a compound leaf developmental regulatory gene *MsPALM1* of *Medicago sativa;* wherein, one strand in the double-stranded structure of the target site has a structure of NGG, wherein N represents any one of bases A, T, C, G;
Step (2), According to the nucleotide sequence of the target site, constructing a binary expression vector MsCRISPR/Cas9::*PALM1* which is used for transforming *Medicago sativa* by *Agrobacterium tumefaciens* and further editing the *MsPALM1* gene of *Medicago sativa;* the MsCRISPR/Cas9*::PALM1* vector comprises an sgRNA expression frame and a *Cas9* nuclease expression frame, the sgRNA expression frame comprises the guide sequence;
Step (3), Introducing the binary expression vector MsCRISPR/Cas9*::PALM1* into *Medicago sativa* cells, so that the sgRNA expression frame and the Cas9 nuclease expression frame are co-expressed in *Medicago sativa* cells; cutting the target site in the double-strand of *MsPALM1* gene, inducing the DNA repairing function of *Medicago sativa* cells themselves; randomly inserting or deleting bases at target sites, resulting in frameshift mutations, so as to cause a loss-of-function mutation in the *MsPALM1* gene of cells;
Step (4), Regenerating plants from the *Medicago sativa* cells in step 3;
Step (5), Performing PCR amplification on the DNA segment comprising the target site of *MsPALM1* gene in the regenerated plants obtained from step 4, and then detecting by restriction endonuclease recognition and/or targeted deep sequencing;
Step (6), Selecting the regenerated plants carrying four alleles with simultaneous loss-of-function mutation for phenotypic identification, observing the compound leaf phenotype of the regenerated plants, and selecting plants with more than 3 leaflets in all the compound leaves as the generated multileaflet *Medicago sativa* material.

Preferably, one strand in the double-stranded structure of the target site has a structure of 5'-G(N)x-NGG-3', wherein, (N)x represents a sequence of x bases {N1, N2••••••Nx}, and each of N1, N2······Nx represents any one of A, G, C, T. X is generally 18 or 19.

Preferably, the sgRNA expression frame can be expressed in *Medicago sativa* cells and its nucleotide sequence is as shown in Seq ID NO. 1; the *Cas9* nuclease expression frame can be expressed in *Medicago sativa* cells and its nucleotide sequence is as shown in Seq ID NO. 2. That is, on the other hand, the present disclosure also provides such a recombinant vector.

Preferably, the sgRNA expression frame comprises: a MtU6 promoter from *Medicago truncatula,* its nucleotide sequence is as shown in positions 1 to 500 of Seq ID NO. 1; a target site with a structural feature of G(N)x and an artificially synthesized sgRNA skeleton sequence, their nucleotide sequences are as shown in positions 501 to 606 of Seq ID NO. 1; and a Poly-T terminator, its nucleotide sequence is as shown in positions 607 to 615 of Seq ID NO. 1,

The *Cas9* nuclease expression frame comprises: a CaMV 35S promoter, its nucleotide sequence is as shown in positions 1 to 345 of Seq ID NO. 2; a *Cas9* coding sequence, as shown in positions 487 to 4756 of Seq ID NO. 2; and a tNOS terminator, as shown in positions 4794 to 5046 of Seq ID NO. 2.

Preferably, the sgRNA expression frame comprises a CRISPR RNA sequence, which has G(N)x in 5'-G(N)x-NGG-3' of the target site or a complementary sequence thereof.

Preferably, the G(N)x in 5'-G(N)x-NGG-3' of the target site or the complementary sequence thereof comprises a sequence of 5'-GGAGACGAGCACGGTCGCGG-3', which is a reverse complementary sequence of the nucleotide sequence 5'-CCGCGACCGTGCTCGTCTCC-3' at the positions 323-343 after the translation initiation codon ATG in the single exon of *MsPALM1* gene.

On the selected exon of *MsPALM1* gene, there are totally 19 fragments with the structure of 5'-G(N)x-NGG-3' which can be selected as the target sites.

The loss-of-function mutation mentioned in step 6 means the occurrence of terminators or reading frame shifts at the target sites of normal *MsPALM1* coding sequences.

The Cas9 nuclease expression frame is located in the same vector comprising the sgRNA expression frame.

In step 3, the obtained recombinant vector is introduced into *Medicago sativa* cells, so that the cells contain sgRNA of the target site and Cas9 nuclease in step 2 simultaneously. Under the combined action of sgRNA and Cas9 nuclease, the double-stranded target site of *MsPALM1* gene is cut, additionally in combination with the DNA repairing function of the *Medicago sativa* cells themselves, random insertion and/or random deletion of *MsPALM1* gene target site can be realized finally in the cells.

In the method, the recombinant vector is introduced into *Medicago sativa* cells by the stable transformation of *Medicago sativa* callus by *Agrobacterium.* During introducing the obtained recombinant vector into the *Medicago sativa* cells, the recombinant vector is introduced into the genomic DNA of *Medicago sativa* by an *Agrobacterium-mediated* method, so the target site of *Medicago sativa* is cut when transgenes are expressed.

In the present disclosure, the method of regenerating plants is to obtain plants through tissue culture of cells or tissues.

In step 5, the DNA fragments comprising the target site of *MsPALM1* gene in the regenerated plants can be cloned by a PCR method with the genome DNA as template, and restriction endonuclease digestion and/or targeted deep sequencing are performed on the amplified products. The PCR method with the genome DNA as template is as below: against the genome region comprising the target site, designing site-specific primers; with the genome DNA of the regenerated plants as the template, amplifying the genome region comprising the target site. The restriction endonuclease digestion on the amplification products means that separating the amplification products by means of agarose gel electrophoresis, then recovering the DNA fragments comprising the target site, digesting the recovered DNA fragments comprising the target site with restriction endonuclease, and performing the agarose gel electrophoresis again; the resulting bands that cannot be digested with restriction endonuclease are the bands that mutate at the target sites. The targeted deep sequencing means that ligating the bands that cannot be digested with restriction endonuclease to the pMD19-T vector and then performing the deep sequencing, or directly ligating the PCR products to the pMD19-T vector and then performing the deep sequencing.

The four *MsPALM1* allelic genes with simultaneous loss-of-function mutation means that none of the recovered DNA fragments comprising the target site can be digested with the restriction endonuclease, and the sequencing results show that four loss-of-function mutation sequences occurred at the target site of *MsPALM1* gene, but there were no wild-type sequences occurred; or, the sequencing results of PCR products show that four loss-of-function mutation sequences occurred at the target site of *MsPALM1* gene, but there were no wild-type sequences occurred.

Wherein, the loss-of-function mutation sequence means the occurrence of terminators or reading frame shifts at the target site of normal *MsPALM1* coding sequences.

Further, the target site comprises one BstUI restriction endonuclease recognition site.

Further, the present disclosure also provides an application of artificial site-directed mutants of the compound leaf developmental regulatory gene *MsPALM1* of *Medicago sativa* in *Medicago sativa* breeding and genome editing breeding.

The breeding method of the present disclosure can be used to produce multileaflet *Medicago sativa* varieties quickly in a short time period, with significant effects.

In particular, compared with traditional breeding methods, the present method has the following benefits:
① Short breeding period, the whole directional production process of the materials can be completed within 7 months, while the traditional hybridization method requires at least 3 to 5 years.
② Only one gene in the recipient varieties is changed, the obtained materials are changed to pentatrinate compound leaves with other agronomic trait unchanged; while the traditional hybridization method may introduce other genes linked with *MsPALM1,* which may influence the agronomic trait of the recipient varieties.
③ In strictly cross-pollinated autotetraploid *Medicago sativa,* a stable genetic homozygous mutant with all the four alleles mutated can be quickly obtained; However, it is very difficult to obtain a stable genetic homozygous mutant by the traditional hybridization method.

### BRIEF DESCRIPTION OF THE DRAWINGS

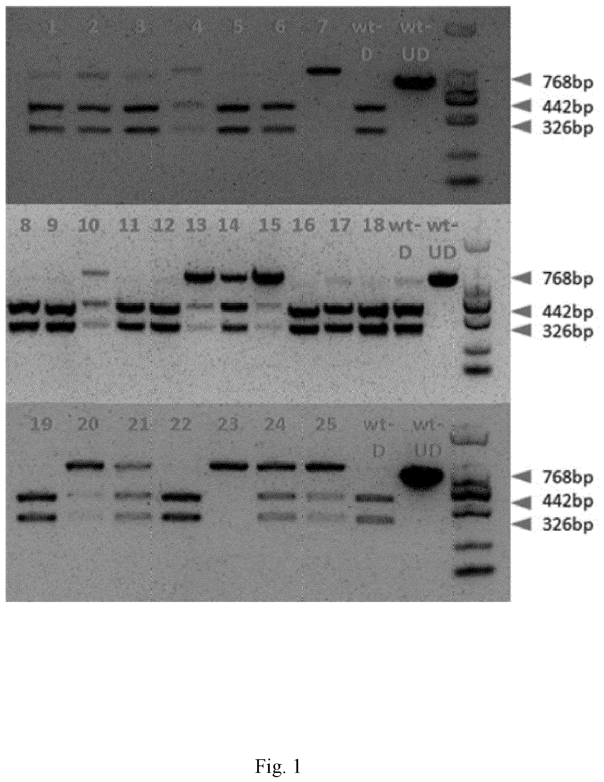

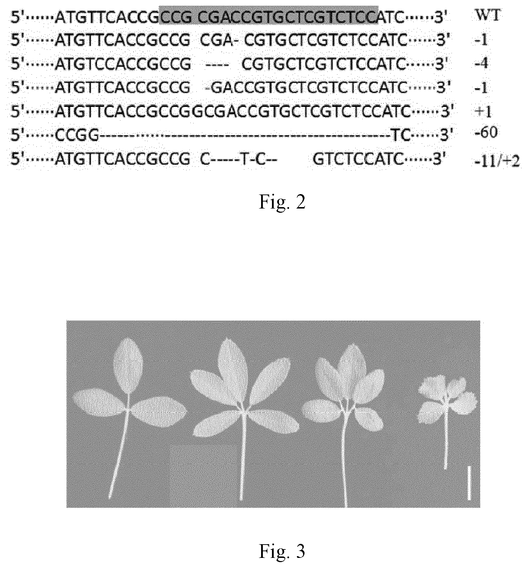

Fig. 1 shows the agarose gel electrophoresis results of PCR-RE analyses for screening *PALM1* mutants out from the regenerated plants that are generated by transforming *Medicago sativa* with the CRISPR/Cas9 expression vector MsCRISPR/Cas9::*PALM1* containing the target site of *PALM1* gene using *Agrobacterium tumefaciens*; Fig. 2 shows the partial sequencing results of PCR products of *PALM1* gene of *Medicago sativa* wild-type and mutant-type plants;

Fig. 3 shows the leaf phenotype of wild-type and mutant-type plants obtained by transforming *Medicago sativa* with the CRISPR/Cas9 expression vector containing the target site of *PALM1* gene using *Agrobacterium tumefaciens.*

### DETAILED DESCRIPTION

The test methods used in the following examples are all conventional methods, unless otherwise specifically noted.

The materials and reagents used in the following examples are all commercially available, unless otherwise specifically noted.

The breeding method employed in one example of the present disclosure will be described below.

First. Preparation of a recombinant vector for editing the *Medicago sativa MsPALM1* gene
1.1. Selecting the reverse complementary sequence, 5'-GGAGACGAGCACGGTCGCGG-3', of the nucleotide sequence 5'-CCGCGACCGTGCTCGTCTCC-3' at the positions 323-343 after the translation initiation codon ATG in the single exon of *MsPALM1* gene (HM038483) of *Medicago sativa* as the target site, and after the sequence was a PAM sequence "CGG". The sequence comprised one BstUI restriction endonuclease recognition site.
1.2. Synthesizing a forward oligonucleotide strand (MsPA1_1F) and a complementary reverse oligonucleotide strand (MsPA1_1R) according to the selected target site (BGI),
   The specific sequences were:
   MsPA1_1F:TTTGGAGACGAGCACGGTCGCGG
   MsPA1_1R:AAACCCGCGACCGTGCTCGTCTC

   Wherein, the non-underlined parts were the sequence from the target site or its complementary sequence without NGG, and the underlined parts were the cohesive ends for ligating the vector.
1.3. After annealing, the two strands MsPA1_1F and MsPA1_1R were annealed to form a double-stranded DNA with cohesive ends, which was used as the insertion fragment for constructing the recombinant vector.
1.4. Digesting the *Medicago sativa* CRISPR/Cas9 binary expression vector MsCRISPR/Cas9 comprising an sgRNA expression frame (its nucleotide sequence was as shown in Seq ID NO. 1) that can be expressed in *Medicago sativa* cells and a *Cas9* nuclease expression frame (its nucleotide sequence was as shown in Seq ID NO. 2) that can be expressed in *Medicago sativa* cells with AarI endonuclease (NEB Co.) at 37°C; cutting the *Medicago sativa* CRISPR/Cas9 expression vector with AarI endonuclease for 8 hours; inactivating the digestion system at 65°C for 10 minutes, as the backbone fragments for constructing the recombinant vector.
1.5. Ligating the MsCRISPR/Cas9 vector backbone fragments and the insertion fragments with T4 DNA ligase (NEB Co.) to construct a vector MsCRISPR/Cas9::*PALM1* for *MsPALM1* editing, and then transferring into the *Escherichia coli.* After verification by sequencing, the positive transformants were extracted to form a recombinant vector MsCRISPR/Cas9::*PALM1* for genome editing of *Medicago sativa MsPALM1* gene.

Second. Genome editing of *Medicago sativa MsPALM1* gene editing by stably transformation with *Agrobacterium* and acquisition of multileaflet *Medicago sativa* materials.
2.1. The obtained recombinant vector MsCRISPR/Cas9::*PALM1* for genome editing of *Medicago sativa MsPALM1* gene was transformed into *Agrobacterium tumefaciens* EHA105 (preserved by Guangdong Sanjie Forage Biotech Co., Ltd.) by a freezing-thawing method, to obtain positive clones.
2.2. Taking mature seeds of *Medicago sativa* varieties, they were washed with sterile water for 5 times, and soaked with 75% alcohol for 1 min. After discarding the alcohol, the seeds were washed with sterile water for 5 times, soaked with 0.1% mercuric chloride solution for 20 min, and washed with sterile water for 5 times. The seeds were inoculated on the germinating medium and cultured in light at 26-28°C for 7 days. When two cotyledons were just opened, the cotyledons and hypocotyls were cut for *Agrobacterium* transformation.
2.3. The *Agrobacterium tumefaciens* into which the recombinant expression vector was transferred was subjected to the genetic transformation of *Medicago sativa,* the specific transformation operations were as below:
   1. Germination of *Medicago sativa* seeds: Plump and well-colored *Medicago sativa* seeds were selected, soaked with 75% alcohol for 2 minutes, washed with sterile water for two times, 1 minute for each time; then soaked with 0.1% mercuric chloride and shaken by hands for 10 minutes, and washed with sterile water for five times. The sterilized seeds were spread out in a large sterile culture dish containing filter papers, air-dried, inoculated on a MS solid medium, and germinated in an illuminated incubator or culture room for 7-14 days;
   2. Induction of callus: Cotyledons and hypocotyls of the germinated seedlings were cut into small pieces with a sterile scalpel and inoculated in a callus induction medium, and cultured in an incubator or culture room in dark at 25±1°C for 3 days. The ingredients of the callus induction medium were: SH basal medium + 2 mg/L 2,4-D + 0.2 mg/L KT + 0.3 mg/L casein hydrolysate + 30 g/L sucrose + 8 g/L agar;
   3. Infection: 1-2 days in advance, the prepared *agrobacterium tumefaciens* strains were inoculated in 50-100 mL YM liquid medium and cultured at 28°C on a shaker at 200 r/min until the OD value 260/280 was between 0.5-0.8; the bacteria solution was transferred into a 50 mL sterile centrifuge tube, and centrifuged at 4°C in a centrifuge at 4000 r/min for 12 minutes; the centrifuge tube was taken out, the supernatant was discarded, and a resuspension solution was added for resuspension until the OD value 260/280 was between 0.5-0.8; Corresponding volume of acetosyringone at 100 µmol/mL was added according to adding 100 µmol acetosyringone per liter, and the resuspension solution was MS liquid medium (MS + 30 g/L sucrose); The explants induced in step 2 were collected into a 100 mL sterile triangular flask with a breathable and plastic sealing membrane, into which was poured a suitable amount of the resuspended bacteria solution (as long as covering the materials); The triangular flask was sealed with its own sealing membrane, and evacuated in a vacuum pump to 0.5 kpa for totally 1 h, during which the flask was shaken gently every 15 min; The triangular flask was taken out and shaken at 28°C on a shaker at 120 r/min for 1 h; All the bacteria solution was poured out, and the materials were spread out in a sterile culture dish containing filter papers to dry out;
   4. Co-cultivation: The dried infected materials were inoculated in a co-cultivation medium (spreading a piece of sterile filter paper in the medium); the ingredients of the co-cultivation medium were: MS basal medium + 2 mg/L 2,4-D + 0.2 mg/L KT + 30 g/L sucrose + 8 g/L agar + 100 µmol/L acetosyringone. They were cultivated in dark in an incubator or in a culture room at 25±1°C for 3 days.
   5. Screening: The co-cultivated materials were inoculated in a screening medium; the ingredients of the screening medium were: SH basal medium + 2 mg/L 2,4-D + 0.2 mg/L KT + 30 g/L sucrose + 8 g/L agar + 250 mg/L cefotaxime + 250 mg/L carbenicillin + 15 mg/L hygromycin; they were cultivated in light in an incubator or in a culture room at 25±1°C for 30-60 days;
   6. Differentiation: The recovered materials were transferred into a differentiation medium; the ingredients of the differentiation medium were: UM basal medium + 2 g/L casein hydrolysate + 0.4 mg/L KT + 30 g/L sucrose + 8 g/L agar + 250 mg/L cefotaxime + 5 mg/L hygromycin; they were cultivated in light in an incubator or in a culture room at 25±1°C for 15-30 days;
   7. Rooting: The differentiated sprouts of 1-3 cm were transferred into a rooting medium; the ingredients of the rooting medium were: MS basal medium + 1 mg/L IBA + 15 g/L sucrose + 8 g/L agar + 250 mg/L cefotaxime.
      25 regenerated *Medicago sativa* plants were obtained totally.
      2.4. The genome DNA was extracted from the obtained 25 transgene *Medicago sativa* plants containing the recombinant vector targeting the *Medicago sativa MsPALM1* gene by means of a plant genome extraction mini kit (Tiangen Biotech Co., Ltd). With the DNA as the template, the sequence containing a target site was subjected to PCR amplification by using an ExTaq DNA polymerase (Takara Co.), wherein the primers used for PCR amplification were:
         MsPA1 genome F:AATTTCATCCCCCACCCCATTA
         MsPA1 genome R:TTCTCCACACACTGAAAAAGAGAGA
      2.5 The obtained PCR amplification fragments were digested with BstUI restriction endonuclease (NEB Co.) to screen the mutants. The digestion results showed that, in the 25 tested plants, 13 plants contained mutations in the *MsPALM1* gene, with a mutation efficiency of 52%, the results were as shown in Fig. 1; wherein there were 3 plants which were regenerated lines carrying four *MsPALM1* allelic genes with simultaneous loss-of-function mutation, the efficiency of simultaneous allele loss-of-function mutation was 12%.
      2.6. Bands which cannot be digested with BstUI restriction endonuclease were cloned by TA to ligate with the pMD19-T vector (Takara Co) and transformed into *Escherichia coli* DH5α (preserved by Guangdong Sanjie Forage Biotech Co., Ltd.); 10 monoclones were selected and sequenced by using M13F universal primers, and the mutations at the target sites were analyzed. The sequencing results showed that, there were mutations at the target sites in all the mutated plants obtained from the screening. Partial results were as shown in Fig. 2 (The shaded part of the image, that was, CCGCGACCGTGCTCGTCTCC starting from position 10 was the target site for editing), the forms of mutation included the insertion and/or deletion of bases.
      2.7. The compound leaf phenotypes of the regenerated plants carrying four *MsPALM1* allelic genes with simultaneous loss-of-function mutation were observed, wherein the compound leaves of 3 *Medicago sativa* plants showed pentatrinate compound leaves phenotypes significantly, indicating that the 3 transgene lines carrying four *MsPALM1* allelic genes with simultaneous loss-of-function mutation were the directed produced multileaflet *Medicago sativa* materials, see Fig. 3.

Compared with traditional breeding methods, the present method has the following benefits:
① Short breeding period, the whole directional production process of the materials can be completed within 7 months, while the traditional hybridization method requires at least 3 to 5 years.
② Only one gene in the recipient varieties is changed, the obtained materials are changed to pentatrinate compound leaves with other agronomic trait unchanged; while the traditional hybridization method may introduce other genes linked with *MsPALM1,* which may influence the agronomic trait of the recipient varieties.
③ In strictly cross-pollinated autotetraploid *Medicago sativa,* a stable genetic homozygous mutant with all the four alleles mutated can be quickly obtained; However, it is very difficult to obtain a stable genetic homozygous mutant by the traditional hybridization method.

## Claims

1. A method of obtaining multileaflet *Medicago sativa* materials by means of *MsPALM1* artificial site-directed mutants, wherein, the method comprises the following steps:
Step (1), Selecting a target site in an exon region of a compound leaf developmental regulatory gene *MsPALM1* of *Medicago sativa;* wherein, one strand in the double-stranded structure of the target site has a structure of NGG, wherein N represents any one of bases A, T, C, G;
Step (2), According to the nucleotide sequence of the target site, constructing a binary expression vector MsCRISPR/Cas9::*PALM1* which is used for transforming *Medicago sativa* by *Agrobacterium tumefaciens* and further editing the *MsPALM1* gene of *Medicago sativa,* the binary expression vector MsCRISPR/Cas9::*PALM1* comprises an sgRNA expression frame and a Cas9 nuclease expression frame, the sgRNA expression frame comprises the *MsPALM1* target site;
Step (3), Introducing the binary expression vector MsCRISPR/Cas9::*PALM1* after being transformed by *Agrobacterium tumefaciens* into *Medicago sativa* cells, so that the sgRNA expression frame and the *Cas9* nuclease expression frame are co-expressed in *Medicago sativa* cells; cutting the target site in the double-strand of *MsPALM1* gene, inducing the DNA repairing function of *Medicago sativa* cells themselves; randomly inserting or deleting bases at target sites, to cause a loss-of-function mutation in the *MsPALM1* gene of cells;
Step (4), Regenerating plants from the *Medicago sativa* cells in step 3;
Step (5), Performing PCR amplification on the DNA segment comprising the target site of *MsPALM1* gene in the regenerated plants obtained from step 4, and then detecting by restriction endonuclease digestion and/or targeted deep sequencing;
Step (6), Selecting the regenerated plants carrying four alleles with simultaneous loss-of-function mutation for phenotypic identification, observing the compound leaf phenotype of the regenerated plants, and selecting plants with more than 3 leaflets in all the compound leaves as the generated multileaflet *Medicago sativa* materials.

2. The method of obtaining multileaflet *Medicago sativa* materials by means of *MsPALM1* artificial site-directed mutants according to claim 1, wherein, one strand in the double-stranded structure of the target site has a structure of 5'-G(N)x-NGG-3', wherein (N)x represents a sequence of x bases {N1, N2••••••Nx}, and each of N1, N2••••••Nx represents any one of A, G, C, T.

3. The method of obtaining multileaflet *Medicago sativa* materials by means of *MsPALM1* artificial site-directed mutants according to claim 1, wherein, the sgRNA expression frame can be expressed in *Medicago sativa* cells and its nucleotide sequence is as shown in Seq ID NO. 1.

4. The method of obtaining multileaflet *Medicago sativa* materials by means of *MsPALM1* artificial site-directed mutants according to claim 1, wherein, the *Cas9* nuclease expression frame can be expressed in *Medicago sativa* cells and its nucleotide sequence is as shown in Seq ID NO. 2.

5. The method of obtaining multileaflet *Medicago sativa* materials by means of *MsPALM1* artificial site-directed mutants according to claim 2, wherein, the sgRNA expression frame comprises: a MtU6 promoter from *Medicago truncatula,* its nucleotide sequence is as shown in positions 1 to 500 of Seq ID NO. 1; a target site with a structural feature of G(N)x and an artificially synthesized sgRNA skeleton sequence, their nucleotide sequences are as shown in positions 501 to 606 of Seq ID NO. 1; and a Poly-T terminator, its nucleotide sequence is as shown in positions 607 to 615 of Seq ID NO. 1,
the *Cas9* nuclease expression frame comprises: a CaMV 35S promoter, its nucleotide sequence is as shown in positions 1 to 345 of Seq ID NO. 2; a *Cas9* coding sequence, as shown in positions 487 to 4756 of Seq ID NO. 2; and a tNOS terminator, as shown in positions 4794 to 5046 of Seq ID NO. 2.

6. The method of obtaining multileaflet *Medicago sativa* materials by means of *MsPALM1* artificial site-directed mutants according to claim 3, wherein, the sgRNA expression frame comprises a CRISPR RNA sequence, which has G(N)x in 5'-G(N)x-NGG-3' of the target site or a complementary sequence thereof.

7. The method of obtaining multileaflet *Medicago sativa* materials by means of *MsPALM1* artificial site-directed mutants according to claims 2 or 6, wherein, the G(N)x in 5'-G(N)x-NGG-3' of the target site or the complementary sequence thereof comprises a sequence of 5'-GGAGACGAGCACGGTCGCGG-3', which is a reverse complementary sequence of the nucleotide sequence 5'-CCGCGACCGTGCTCGTCTCC-3' at the positions 323-343 after the translation initiation codon ATG in the single exon of *MsPALM1 gene.Medicago sativa*

8. The method of obtaining multileaf *Medicago sativa* material by means of *MsPALM1* artificial site-directed mutant according to claim 7, wherein the target comprises one BstUI restriction endonuclease site.*Medicago sativa*

9. An application of the *MsPALM1* artificial site-directed mutant of any one of claim 1-8 in *Medicago sativa* breeding and *Medicago sativaMedicago sativaMedicago sativaMedicago sativaMedicago sativaMedicago sativaMedicago sativaMedicago sativaMedicago sativa*
